# EUROPEAN PATENT SPECIFICATION

(11) **EP 2 284 188 B1**
(45) Date of publication and mention of the grant of the patent: **04.07.2012**
(21) Application number: 09167004.2
(22) Date of filing: 31.07.2009
(51) Int. Cl.: C07K 14/47, C07K 17/08, G01N 33/564, G01N 33/68

(54) **Detection of anti-ribosomal P protein antibodies by means of synthetic peptides**
Erkennung von anti-ribosomalen P-Protein-Antikörpern durch synthetische Peptide
Détection d'anticorps de protéine P anti-ribosomale au moyen de peptides synthétiques

(43) Date of publication of application: 16.02.2011
(73) Proprietor: Toscana Biomarkers S.r.l., 53100 Siena (IT)
(72) Inventor: Pratesi, Federico, 53100 Siena (IT); Alcaro, Maria Claudia, 53100 Siena (IT); Chelli, Mario, 53100 Siena (IT); Fantini, Paolo, 53100 Siena (IT); Lolli, Francesco, 53100 Siena (IT); Paolini, Ilaria, 53100 Siena (IT); Papini, Anna Maria, 53100 Siena (IT); Rovero, Paolo, 53100 Siena (IT); Migliorini, Paola, 53100 Siena (IT)
(74) Representative: Capasso, Olga

(56) References cited:
- US-A- 4 865 970
- CAPONI L ET AL: "Autoantibodies directed against ribosomal P proteins: use of a multiple antigen peptide as the coating agent in ELISA" JOURNAL OF IMMUNOLOGICAL METHODS, ELSEVIER SCIENCE PUBLISHERS B.V.,AMSTERDAM, NL, vol. 179, no. 2, 27 February 1995 (1995-02-27), pages 193-202, XP004021084 ISSN: 0022-1759
- KISS EMESE ET AL: "Are anti-ribosomal P protein antibodies relevant in systemic lupus erythematosus?" CLINICAL REVIEWS IN ALLERGY & IMMUNOLOGY FEB 2007, vol. 32, no. 1, February 2007 (2007-02), pages 37-46, XP002551258 ISSN: 1080-0549
- MARSDEN H S ET AL: "Advantages of branched peptides in serodiagnosis" JOURNAL OF IMMUNOLOGICAL METHODS, ELSEVIER SCIENCE PUBLISHERS B.V.,AMSTERDAM, NL, vol. 147, 1 January 1992 (1992-01-01), pages 65-72, XP002131246 ISSN: 0022-1759
- TAM AND F ZAVALA J P: "Multipel antigen peptide" JOURNAL OF IMMUNOLOGICAL METHODS, ELSEVIER SCIENCE PUBLISHERS B.V.,AMSTERDAM, NL, vol. 124, 1 January 1989 (1989-01-01), pages 53-61, XP002131245 ISSN: 0022-1759

## Description

### Field of invention

The present invention refers to methods for the identification of diagnostic antibodies in autoimmune diseases such as Systemic Lupus Erythematosus (SLE), and therefore useful tools for diagnosis or therapeutic treatment of SLE.

### State of the art

Systemic lupus erythematosus (SLE) is an autoimmune disease characterized by the presence of antibodies against nuclear components and against ribosomal proteins. It has been shown that antibodies against the three protein components of the 60S ribosomal subunit, called P0, P1, and P2, are specifically associated with SLE and more frequently detected in active disease, especially in patients with nephritis [K.B. Elkon et al., J. Exp. Med. 1985, 162, 459; A.M. Francoeur et al., J. Immunol. 1985, 135, 2378; E. Bonfa et al., N. Engl. J. Med. 1987, 317, 265]. The anti-ribosomal P antibodies were identified in sera from SLE patients by immunoblotting analysis on ribosomal extracts.

It has been verified that the antigenic determinant of ribosomal P proteins is located in the C-terminal region of the ribosomal protein eL12 of the organism *Artemia Salina* [K. Elkon e/ al., Proc. Nat. Acad. Sci. USA 1986, 83, 7419]. A method based on the use of a peptide comprising the last 22 amino acids (C-22: H-Lys-Lys-Glu-Glu-Lys-Lys-Glu-Glu-Ser-Glu-Glu-Glu-Asp-Glu-Asp-Met-Gly-Phe-Gly-Leu-Phe-Asp-OH, SEQ ID No. 1) [N. Brot et al., US4865970] has been demonstrated to detect SLE specific autoantibodies in about 10-20% of patients sera [K. Elkon et al., Proc. Nat. Acad. Sci. USA 1988, 85, 5186; F.B. Karassa et al., Arthritis Rheum. 2006, 54, 312]. The sensitivity of the ELISA based on the peptide C-22 was further improved using the four-branched multiple antigenic peptide (MAP) of C-22 [B.F. Bruner et al., Ann. N. Y. Acad. Sci. 2005, 1051, 390] or of the last C-terminal 13 amino acids [L. Caponi et al., J. Immunol. Methods 1995, 179, 193]. Nevertheless, some studies have been performed to identify conformational epitopes [E. Koren et al., J. Clin. Invest. 1992, 89, 1236; M Mahler et al., Clin. Vaccine Immunol. 2006, 13, 77] or different epitopic regions of ribosomal P proteins [N. Fabien et al., J. Autoimmun. 1999, 13, 103]. A peptide scan through the complete amino acid sequences of human P0, P1, and P2 confirmed the reported immunodominance of the C-terminal epitope [M. Mahler et al., J. Mol. Med. 2003, 81, 194]. No central or N-terminal peptides were demonstrated to be more active than C-22 in the test conditions. A further epitope mapping, performed on the full sequence of human P0 ribosomal protein, demonstrated the presence of other antigenic regions less active than C-22 [B.F. Bruner et al., Ann. N. Y. Acad. Sci. 2005, 1051, 390].

### Description of the invention

A technical problem still unsolved by the above prior art is the provision of specific and reliable diagnostic tools for auto-immune diseases, specifically SLE.

The present invention refers to the use of peptides derived from the N-terminal portion of the P1 ribosomal protein, that surprisingly detect specific autoantibodies in SLE patients sera. In addition, another embodiment of the present invention is the development of a more sensitive peptide-based method of detecting SLE. The peptides of the present invention, also conjugated to a resin, can be used for therapeutic treatments of SLE patiens.

Authors performed a complete epitope mapping of the sequences of human P1 and P2 proteins. 14 peptides of 20 amino acids were synthesized with an overlapping of 5 amino acids comprising the N-terminal regions. The peptides were obtained as pure products (purity >95%) and were tested on 25 sera of SLE patients and 68 healthy blood donors.

It has been surprisingly found, and is a subject of the present invention, that a peptide derived from the N-terminal region of ribosomal P1 protein, of 10-20 amino acids, has a very efficient role in the recognition of autoantibodies typical of autoimmune diseases and is therefore an useful tool for diagnosis or therapeutic treatment of SLE.

It is an object of the invention a multimeric branched peptide consisting in:
- a MAP nucleus structure;
- a linear peptide consisting of an amino acid sequence of at least 10 aa comprised in the sequence of SEQ ID No. 2, bonded through a carbamido link to each of amino terminal residues of the MAP nucleus structure, wherein the linear peptides are equal or different each others.

According to the invention a MAP nucleus structure is: 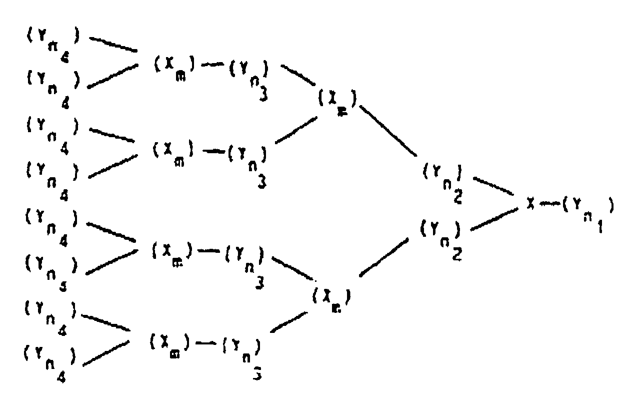 wherein X is an amino acid having at least two amino functional residues, Y is an amino acid selected from the group of alanine, glycine or arginine, m is 0 or 1, n₁ n₂ n₃ n₄ are integer numbers comprised between 0 and 10, and wherein bond are carbamido bonds.

As it is known from the scientific literature [J.P. Tam, Proc. Natl. Acad. Sci. USA 1988, 85, 5409], the MAP structure is characterized by an immunological inert amino acid core, generally a branched core of lysine or of lysine and alanine, to which a number of identical copies of the antigen sequence of interest, generally 4 or 8 copies, are bound. The expression "immunological inert core" means that, when MAPs are used as immunogens, they don't induce the production of antibodies directed against the central amino acid core.

Preferably the multimeric branched peptide is a tetrameric branched peptide and the linear peptides consist of an amino acid sequence belonging to the following group: aa 1-20 of Seq. ID No. 2; aa 1-10 of Seq. ID No. 2; aa 6-16 of Seq. ID No. 2; aa 11-20 of Seq. ID No. 2, as in the below scheme:

| | | |
|---|---|---|
| 1 | Seq. ID No. 2 | |
| 2 | aa 1-10 Seq. ID No. 2 | Leu-Ile-Lys-Ala-Ala-Gly-Val-Asn-Val-Glu |
| 3 | aa 6-16 Seq. ID No. 2 | Gly-Val-Asn-Val-Glu-Pro-Phe-Trp-Pro-Gly-Leu |
| 4 | aa 11-20 Seq. ID No, 2 | Pro-Phe-Trp-Pro-Gly-Leu-Phe-Ala-Lys-Ala |

Preferably, the MAP comprises four identical copies of the above reported preferred peptides. In details, the following MAPs containing the amino acid sequences as above are preferred:

| | |
|---|---|
| 5 | |
| 6 | (Leu-Ile-Lys-Ala-Ala-Gly-Val-Asn-Val-Glu)₄-Lys₂-Lys-betaAla |
| 7 | (Gly-Val-Asn-Val-Glu-Pro-Phe-Trp-Pro-Gly-Leu)₄-Lys₂-Lys-betaAla |
| 8 | (Pro-Phe-Trp-Pro-Gly-Leu-Phe-Ala-Lys-Ala)₄-Lys₂-Lys-betaAla |

It is another object of the invention a peptide composition comprising at least one multimeric branched peptide as above disclosed and a further tetrameric branched peptide comprising a MAP nucleus structure and four linear peptides having the amino acid sequence 10-22 of SEQ ld No. 1.

Another aspect of the present invention is the development of a diagnostic method to increase the specificity of the immunological assays. The preferred diagnostic method is performed testing the peptides 1-8 and the four-branched MAP of the last C-terminal 13 amino acids of the ribosomal protein eL12 of the organism *Artemia Salina* (Seq ID. No. 1: Glu-Glu-Glu-Asp-Glu-Asp-Met-Gly-Phe-Gly-Leu-Phe-Asp).

The multimeric branched peptide in the MAP format has the following structure:

| | |
|---|---|
| 9 | (Glu-Glu-Glu-Asp-Glu-Asp-Met-Gly-Phe-Gly-Leu-Phe-Asp)₄-Lys₂-Lys-betaAla |

The combined use of the two different antigenic regions allowed to detect the presence and/or measuring of the levels of anti-ribosomal P protein antibodies in samples of biological fluids in an increased number of SLE patients.

The immunological assay based on the preferred peptides comprised in Seq Id No. 1 and on the tetrameric MAP branched of formula 9 can be performed either mixing them during the coating step or using them in parallel, as in multiplexed experiments.

It is another object of the invention the use of a peptide consisting of an amino acid sequence of at least 10 aa of the sequence of SEQ ID No. 2, for the *in vitro* detection of auto-antibodies specific for an auto-immune disease. Preferably said peptide consists of an amino acid sequence belonging to the following group: aa 1-20 of Seq. ID No. 2; aa 1-10 of Seq. ID No. 2; aa 6-16 of Seq. ID No. 2; aa 11-20 of Seq. ID No. 2. The peptides of the inventions may be bound to a solid support.

It is another object of the invention an immunodiagnostic kit for detecting auto-antibodies specific for an auto-immune disease comprising as selective reagent the peptide or the composition as above disclosed. Preferably the auto-immune disease is SLE.

It is another object of the invention a method for detecting auto-antibodies specific for an auto-immune disease in a fluid sample comprising the step of incubating said fluid sample with the peptide or the composition as above disclosed, and detecting bound antibodies. Preferably the auto-immune disease is SLE.

In order to perform the immunological assay, the peptides can be adsorbed or covalently linked or modified with a carrier to bind it to a solid support (e.g. chips, microspheres, gold, polystyrene, reactor vessels or wells, micro-titre plate). In a first step of the assay method, the sample of biological fluid to be analyzed is placed in contact and incubated with the preferred peptide linked on the solid support. Any anti-ribosomal P protein antibodies that are possibly present in the sample are thus specifically bound to the preferred peptide, producing an antigen/antibody complex. The anti-ribosomal P protein antibodies to be detected in the immunoassay are IgG immunoglobulins. The evaluation of the presence and the quantization of the antigen/antibody complex can be performed with a spectroscopic, a piezoelectric, or an electrochemical biosensor.

In a preferred embodiment, the above described method is an ELISA immunological assay in which an indicator antibody, like a anti-human IgG, is conjugated to an enzyme and is added to measure the antibody titer by a spectroscopic transducer.

In another preferred embodiment of the present invention, peptides as above disclosed in free form or bound to appropriate resins, can be used for the treatment of patients affected by SLE as, thanks to their high specificity of antibody recognition, they can be used to selective antibody removal and also immunomodulation of the disease.

### Detailed description of the invention

Examples disclosing the preparation of some peptides according to the invention, are provided in the following for illustrative, non limiting purposes of the invention.

### Example 1. Peptide Synthesis

Peptide sequences were selected after an epitope mapping performed on the full sequence of human ribosomal P1 and P2 proteins. Peptides were synthetized using a Wang resin preloaded with the C-terminal amino acid of the sequence or with the MAP core and following the Fmoc/tBu solid-phase peptide strategy. Fmoc deprotections were carried out in 20 min with 20% piperidine in DMF. Coupling reactions were performed by treating the resin for 45 min with a 0.5 M solution of the Fmoc-protected amino acids and HOBt in DMF (2.5 equiv), a 0.5 M solution of TBTU in DMF (2.5 equiv), and 4 M NMM in DMF (5 equiv). Peptide cleavage from the resin and deprotection of the amino acid side chains were carried out in 3 h with TFA/thioanisole/ethanedithiol/phenol/H₂O (82.5:5:2.5:5:5). The crude products were precipitated with cold Et₂O, centrifuged, and lyophilized. The pure peptides were obtained by HPLC in a purity >95% and characterized by mass spectrometry (ESI-Orbitrap and/or MALDI-TOF).

### Example 2. Antibody detection by ELISA

A peptide, prepared as described in example 1 and diluted in 0.05 M carbonate buffer (pH 9.6) or in PBS buffer (pH 7.4) was adsorbed onto 96-wells microtitre plates for 4 h at r.t. The wells were treated for 1 h at r.t. with FBS (FBS 5% in PBS). The patients serum samples (diluted 1:100) were then loaded onto the plate and allowed to react overnight at +4 °C. After plate washing, an anti human-IgG antibody conjugated to the enzyme alkaline phosphatase was added and incubated for 3 h at r.t. After washings, the alkaline phosphate substrate p-nitrophenyl phosphate was added to the wells. After the quenching of the enzymatic reaction with 1 M NaOH, the absorbance was evaluated at a wavelength of 405 nm.

### Example 3. ELISA based on the contemporary use of peptides 1 and 9

To achieve a highly sensitive immunoassay, an equimolar mixture of peptides 1 and 9 were allowed to adsorb to 96-wells microtitre plates for 4 h at r.t. The test was then performed as described in example 2.

### Results

In total, 25 serum samples of patients suffering from SLE and 68 serum of healthy blood donors were tested by this method. Results are reported in Table 1.

**Table 1. ELISA results of peptides 1 and 5-9 with SLE and healthy donor sera.**

| Peptide | SLE positive sera | healthy donor positive sera |
|---|---|---|
| 1 | 8/25 (32%) | 1/68 (1%) |
| 5 | 4/23 (17%) | 2/27 (7%) |
| 9 | 15/102 (15%) | 1% |

Considering that anti-ribosomal P antibodies are present in 10-20% of SLE patients sera, as object of this patent, peptides recognizing more than 10-20% of SLE patients sera were preferred.

Peptide 1 of formula (I) was able to recognize antibodies in 32% of SLE patients and in only 1% of healthy controls. Peptide 1 and 9 recognized different populations of autoantibodies in SLE sera and the total number of SLE sera positive to peptide 1 and 9 is 17/25 (68%). In particular, among the 13 SLE sera positive to peptide 9, only 4 of serum showed a cross-reactivity with peptide 1. Using the method based on peptide 1 it was possible to detect anti ribosomal antibodies in 3 SLE serum negative to peptide 9.

The results demonstrated the presence of two different subclasses of autoantibodies: one reactive against an N-terminal domain of human P1, and the other one positive to the C-terminal region common to all the three ribosomal P proteins.

The method set up evaluating the SLE sera positive to peptide 1 and 9 has a sensitivity of 68%.

### Example 4. Antibody detection by covalent ELISA

A peptide, prepared as described in example 1, was covalently linked to a 96-wells microtitre plate (carboxy or amine binding plate) using sulfo-N-hydroxysuccinimide and 1-ethyl-3-(3-dimethylaminopropyl)carbodiimide. Peptide coupling took place for 2 h at r.t. The plate was blocked for 1 h with 5% FBS in PBS or with 0.5 M Gly (pH 8). The patients serum samples were diluted 1:100 in FBS buffer and were then loaded onto the plate for 1.5 h at r.t. After plate washings, an anti human-IgG antibody conjugated to the enzyme alkaline phosphatase was added and incubated for 1.5 h at r.t. After washings, the alkaline phosphate substrate p-nitrophenyl phosphate was added to the wells. After the quenching of the enzymatic reaction with 1 M NaOH, the absorbance was evaluated at a wavelength of 405 nm.

### Results

Results for the antibody detection by covalent ELISA for peptides 1-4 are reported in Table 2.

**Table 2. ELISA results of peptides 1-4 with SLE and healthy donor sera.**

| Peptide | SLE positive sera | healthy donor positive sera |
|---|---|---|
| 1 | 4/8 (50%) | 0/6 (0%) |
| 2 | 3/8 (37%) | 0/6 (0%) |
| 3 | 1/8 (12%) | 0/6 (0%) |
| 4 | 1/8 (12%) | 0/6 (0%) |

### Example 5. Conjugation of a peptide to resin for therapeutic uses

A peptide prepared as described in example 1 was conjugated to sepharose resin preactivated with CNBr, according to the usual reaction protocols advised by the manufacturers to obtain a resin-peptide conjugate. The product thus obtained is useful as for example for the preparation of plates for the diagnosis or treatment of patients affected by SLE.

## Claims

1. A multimeric branched peptide consisting in:
- a MAP nucleus structure;
- a linear peptide consisting of an amino acid sequence of at least 10 aa comprised in the sequence of SEQ ID No. 2, bonded through a carbamido link to each of amino terminal residues of the MAP nucleus structure, wherein the linear peptides are equal or different each others.

2. The multimeric branched peptide according to claim 1 being a tetrameric branched peptide and consisting of the amino acid sequence of the linear peptides belonging to the following group: aa 1-20 of Seq. ID No. 2; aa 1-10 of Seq. ID No. 2; aa 6-16 of Seq. ID No. 2; aa 11-20 of Seq. ID No. 2.

3. A peptide composition comprising at least one peptide of claims 1 to 2 and a tetrameric branched peptide comprising a MAP nucleus structure and four linear peptides having the amino acid sequence 10-22 of SEQ Id No. 1.

4. Use of a peptide consisting of an amino acid sequence of at least 10 aa comprised in the sequence of SEQ ID No. 2, for the *in vitro* detection of auto-antibodies specific for an auto-immune disease.

5. Use of the peptide according to claim 4 wherein said peptide consists of an amino acid sequence belonging to the following group: aa 1-20 of Seq. ID No. 2; aa 1-10 of Seq. ID No. 2; aa 6-16 of Seq. ID No. 2; aa 11-20 of Seq. ID No. 2.

6. The peptide according to any of previous claims wherein the peptide is bound to a solid support.

7. An immunodiagnostic kit for detecting auto-antibodies specific for an auto-immune disease comprising as selective reagent the peptide or the composition according to any of previous claims.

8. The immunodiagnostic kit according to claim 7 wherein the auto-immune disease is SLE.

9. A method for detecting auto-antibodies specific for an auto-immune disease in a fluid sample comprising the step of incubating said fluid sample with the peptide or the composition according to any of previous claims 1 to 6, and detecting bound antibodies.

10. The method for detecting autoantibodies according to claim 9 wherein the auto-immune disease is SLE.

11. A peptide according to claims 1 to 3 for medical use.

## Patentansprüche

1. Multimeres verzweigtes Peptid, das aus Folgendem besteht:
- einer MAP-Nukleusstruktur;
- einem linearen Peptid, bestehend aus einer Aminosäuresequenz mit mindestens 10 aa, die in der Sequenz von Seq.-ID Nr. 2 enthalten ist, welches durch eine Carbamido-Bindung mit jedem der aminoterminalen Reste der MAP-Nukleusstruktur verknüpft ist, wobei die linearen Peptide gleich oder untereinander verschieden sind.

2. Multimeres verzweigtes Peptid nach Anspruch 1, das ein tetrameres verzweigtes Peptid ist und aus der Aminosäuresequenz der linearen Peptide besteht, die zu der folgenden Gruppe gehören: aa 1-20 von Seq.-ID Nr. 2; aa 1-10 von Seq.-ID Nr. 2; aa 6-16 von Seq.-ID Nr. 2; aa 11-20 von Seq.-ID Nr. 2.

3. Peptid-Zusammensetzung, die mindestens ein Peptid der Ansprüche 1 bis 2 und ein tetrameres verzweigtes Peptid, das eine MAP-Nukleusstruktur umfasst, und vier lineare Peptide, welche die Aminosäuresequenz 10-22 von Seq.-ID Nr. 1 aufweisen, umfasst.

4. Verwendung eines Peptids, das aus einer Aminosäuresequenz mit mindestens 10 aa besteht, die in der Sequenz von Seq.-ID Nr. 2 enthalten ist, zum Invitro-Nachweis von Autoantikörpern, die für eine Autoimmunerkrankung spezifisch sind.

5. Verwendung des Peptids nach Anspruch 4, wobei das Peptid aus einer Aminosäuresequenz besteht, die zu der folgenden Gruppe gehört: aa 1-20 von Seq.-ID Nr. 2; aa 1-10 von Seq.-ID Nr. 2; aa 6-16 von Seq.-ID Nr. 2; aa 11-20 von Seq.-ID Nr. 2.

6. Peptid nach einem der vorangehenden Ansprüche, wobei das Peptid an einen festen Träger gebunden ist.

7. Immundiagnostisches Kit zum Nachweis von Autoantikörpern, die für eine Autoimmunerkrankung spezifisch sind, das als selektives Reagenz das Peptid oder die Zusammensetzung nach einem der vorangehenden Ansprüche umfasst.

8. Immundiagnostisches Kit nach Anspruch 7, wobei die Autoimmunerkrankung SLE ist.

9. Verfahren zum Nachweisen von Autoantikörpern, die für eine Autoimmunerkrankung spezifisch sind, in einer flüssigen Probe, welches den Schritt der Inkubation der flüssigen Probe mit dem Peptid oder der Zusammensetzung nach einem der vorangehenden Ansprüche 1 bis 6 und den Nachweis gebundener Antikörper umfasst.

10. Verfahren zum Nachweisen von Autoantikörpern nach Anspruch 9, wobei die Autoimmunerkrankung SLE ist.

11. Peptid nach den Ansprüchen 1 bis 3 zur medizinischen Verwendung.

## Revendications

1. Peptide multimère ramifié constitué de :
- une structure de noyau MAP ;
- un peptide linéaire constitué d'une séquence d'acides aminés d'au moins 10 aa comprise dans la séquence de SEQ ID N° 2, lié par une liaison carbamido à chacun des résidus amino terminaux de la structure de noyau MAP, dans lequel les peptides linéaires sont identiques ou différents les uns des autres.

2. Peptide multimère ramifié selon la revendication 1 étant un peptide tétramère ramifié et étant constitué de la séquence d'acides aminés des peptides linéaires appartenant au groupe suivant : aa 1-20 de SEQ ID NO : 2 ; aa 1-10 de SEQ ID NO : 2 ; aa 6-16 de SEQ ID NO : 2 ; aa 11-20 de SEQ ID NO : 2.

3. Composition de peptide comprenant au moins un peptide selon les revendications 1 à 2 et un peptide tétramère ramifié comprenant une structure de noyau MAP et quatre peptides linéaires ayant la séquence d'acides aminés 10-22 de SEQ ID NO : 1.

4. Utilisation d'un peptide constitué d'une séquence d'acides aminés d'au moins 10 aa comprise dans la séquence de SEQ ID N° 2, pour la détection *in vitro* d' auto-anticorps spécifiques d'une maladie auto-immune.

5. Utilisation du peptide selon la revendication 4, dans laquelle ledit peptide est constitué d'une séquence d'acides aminés appartenant au groupe suivant : aa 1-20 de SEQ ID NO : 2 ; aa 1-10 de SEQ ID NO : 2 ; aa 6-16 de SEQ ID NO : 2 ; aa 11-20 de SEQ ID NO : 2.

6. Peptide selon l'une quelconque des revendications précédentes, dans lequel le peptide est lié à un support solide.

7. Kit d'immuno-diagnostic pour la détection d'auto-anticorps spécifiques d'une maladie auto-immune comprenant en tant que réactif sélectif le peptide ou la composition selon l'une quelconque des revendications précédentes.

8. Kit d'immuno-diagnostic selon la revendication 7, dans lequel la maladie auto-immune est le LED.

9. Procédé de détection d'auto-anticorps spécifiques d'une maladie auto-immune dans un échantillon de liquide comprenant l'étape consistant à faire incuber ledit échantillon de liquide avec le peptide ou la composition selon l'une quelconque des revendications 1 à 6, et la détection d'anticorps liés.

10. Procédé de détection d'auto-anticorps selon la revendication 9 dans lequel la maladie auto-immune est le LED.

11. Peptide selon les revendications 1 à 3, destiné à un usage médical.


### SEQUENCE LISTING

<110> TOSCANA BIOMARKERS srl
<120> Detection of anti-ribosomal P protein antibodies by means of synthetic peptides
<130> BE107151
<160> 2
<170> PatentIn version 3.5
<210> 1
   <211> 22
   <212> PRT
   <213> Artemia salina
<400> 1
   Lys Lys Glu Glu Lys Lys Glu Glu Ser Glu Glu Glu Asp Glu Asp Met
   1               5                   10                  15
   Gly Phe Gly Leu Phe Asp
               20
<210> 2
   <211> 20
   <212> PRT
   <213> Homo sapiens
<400> 2
   Leu Ile Lys Ala Ala Gly Val Asn Val Glu Pro Phe Trp Pro Gly Leu
   1               5                   10                  15
   Phe Ala Lys Ala
               20